# EUROPEAN PATENT APPLICATION

(11) **EP 4 413 966 A1**
(43) Date of publication of application: **14.08.2024**
(21) Application number: 22878637.2
(22) Date of filing: 07.10.2022
(51) Int. Cl.: A61K 6/30, A61K 6/60, A61K 6/61, A61K 6/70, A61K 6/76, A61K 6/77, A61K 6/831, A61K 6/836, A61K 6/86

(54) **PASTE-FORM TWO-PART CURABLE COMPOSITION FOR DENTAL USE**

(30) Priority: 08.10.2021 JP 2021166528
(71) Applicant: Kuraray Noritake Dental Inc., Kurashiki-shi, Okayama 710-0801 (JP)
(72) Inventor: TAKEI, Mitsuru, Tainai-shi, Niigata 959-2653 (JP); KAWANA, Mariko, Tainai-shi, Niigata 959-2653 (JP); OKADA, Keishu, Tainai-shi, Niigata 959-2653 (JP)
(74) Representative: D Young & Co LLP
(86) International application number: PCT/JP2022/037732
(87) International publication number: WO 2023/058770

(57) **Abstract**

The present invention provides a paste-form two-pack type dental curable composition having an adequate working time range and superior bond strength to dentin, in addition to showing small variation in working time, small decline in bond strength to dentin, and low risk of paste solidification over extended storage. The present invention relates to a paste-form two-pack type dental curable composition comprising: a first agent that comprises an acidic group-containing polymerizable monomer (A), an acidic group-free polymerizable monomer (B), an organic peroxide (C), a nitrogen atom-containing ligand (D), and a filler (E); and a second agent that comprises an acidic group-free polymerizable monomer (B), a thiourea compound (F), and a filler (E).

## Description

### TECHNICAL FIELD

The present invention relates to paste-form two-pack type dental curable compositions used in applications such as luting of dental prostheses, such as crowns, inlays, and bridges, to tooth structures, and construction of abutments in dental treatments. Specifically, the invention relates to a paste-form two-pack type dental curable composition having an adequate working time range and superior bond strength to dentin, in addition to showing small decline in bond strength to dentin, low risk of paste solidification, and small variation in working time over extended storage.

### BACKGROUND ART

Adhesive materials and restorative filling materials are widely used for the restorative treatment of tooth defects caused by caries, fractures, or other forms of damage. Resin-based dental curable compositions comprising components such as radical polymerizable monomers, polymerization initiators, and fillers are commonly employed as such adhesive materials and restorative filling materials in tooth restoration.

Within such resin-based dental curable compositions, the material used to bond the prosthesis to the tooth structure is called dental resin cement. In the restorative treatment of deep cavities reaching the pulp, the procedure requires the removal of the pulp and the construction of the abutment tooth. The material utilized for this purpose is referred to as composite resin for dental abutment construction. Both dental resin cement and composite resin for dental abutment construction are paste-form compositions, and are typically produced by mixing a polymerizable monomer-containing liquid composition, containing dissolved components such as polymerizable monomers, a polymerization initiator system, and stabilizers, with components such as powdery fillers. These materials are supplied in containers to dentists for use. It is essential for dental curable compositions to maintain certain performance standards during their designated period of use.

Generally, (meth)acrylate is used as a polymerizable monomer incorporated in dental cements and dental abutment construction materials. Polymerizable monomers having acidic groups such as phosphoric acid groups or carboxyl groups are also incorporated to confer dental cements with self-adhesive properties to tooth structures and prostheses.

A redox-type polymerization initiator system with primary components such as an oxidizing agent and a reducing agent is employed to polymerize and cure such dental curable compositions. The oxidizing agent and reducing agent of the polymerization initiator system are separately formulated. For use, the dentist mixes these components-a first agent containing the oxidizing agent and a second agent containing the reducing agent-to cause a redox reaction, creating radicals that drive the polymerization and curing of the composition.

Traditionally, a common choice for the redox-type polymerization initiator system used in dental curable compositions has been a polymerization initiator system that combines benzoyl peroxide and aromatic amine compounds. However, using this initiator system presents an issue of low storage stability in the composition due to the low heat stability of benzoyl peroxide. Specifically, prolonged storage of compositions containing benzoyl peroxide at temperatures higher than room temperature may lead to issues such as reduced curability and premature solidification of the composition before use due to benzoyl peroxide decomposition. Hence, when supplying compositions containing benzoyl peroxide to users like dentists, it is essential to take measures, for example, such as specifying a storage temperature below room temperature or shortening the expiration date. Indeed, there is potential for improvement in terms of usability and quality stability.

Against this backdrop, there have been proposed polymerization initiator systems in which various types of more stable organic peroxides are used as alternative oxidizing agents to benzoyl peroxide (for example, Patent Literatures 1 and 2).

Patent Literature 1 proposes a two-part self-adhesive composition with storage stability. This composition comprises a hydroperoxide compound having at least one hydroperoxide group attached to a tertiary carbon, and a substituted thiourea of a specific structure.

Patent Literature 2 proposes a two-component initiator system comprising a hydroperoxide compound with one or multiple hydroperoxide groups attached to a tertiary carbon, a thiourea derivative, and a soluble copper compound serving as an accelerator within the preparation.

### CITATION LIST

### Patent Literature

Patent Literature 1: JP 2005-8622 A
Patent Literature 2: JP 2007-56020 A

### SUMMARY OF INVENTION

### Technical Problem

The present inventors assessed the compositions described in Patent Literatures 1 and 2, and identified potential for further improvement regarding storage stability, such as the need to shorten the storage duration because the paste, when stored for extended time periods, decreases its curability, potentially leading to prolonged working time and reduced adhesion to dentin, and paste solidification.

It is accordingly an object of the present invention to provide a paste-form two-pack type dental curable composition having an adequate working time range and superior bond strength to dentin, in addition to showing small variation in working time, small decline in bond strength to dentin, and low risk of paste solidification over extended storage.

### Solution to Problem

Specifically, the present invention provides the following.
[1] A paste-form two-pack type dental curable composition comprising:
   a first agent that comprises an acidic group-containing polymerizable monomer (A), an acidic group-free polymerizable monomer (B), an organic peroxide (C), a nitrogen atom-containing ligand (D), and a filler (E); and
   a second agent that comprises an acidic group-free polymerizable monomer (B), a thiourea compound (F), and a filler (E).
[2] The paste-form two-pack type dental curable composition according to [1], wherein the nitrogen atom-containing ligand (D) comprises a ligand having two or more nitrogen atoms within the molecule.
[3] The paste-form two-pack type dental curable composition according to [1] or [2], wherein:
   the nitrogen atom-containing ligand (D) is at least one compound selected from the group consisting of a compound represented by general formula (1), a compound represented by general formula (2), and a polydentate ligand (3) containing a nitrogen-containing heterocyclic ring, and
   the polydentate ligand (3) is a bidentate or higher dentate ligand compound that comprises a heterocyclic ring containing a five-membered or six-membered ring having a nitrogen atom, and has two or more nitrogen atoms within the molecule,

      R₁R₂N-X₁-NR₃R₄ (1)

      wherein R₁ to R₄ each independently represent an optionally substituted alkyl group, and X₁ represents an optionally substituted divalent aliphatic group, 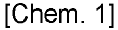 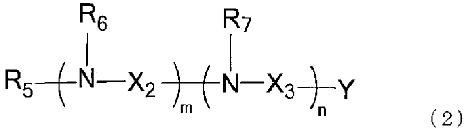 wherein R₅, R₆, and R₇ each independently represent an optionally substituted alkyl group, X₂ and X₃ each independently represent an optionally substituted divalent aliphatic group that may contain an oxygen atom and/or a nitrogen atom, m and n each independently represent an integer of 1 or more, Y represents an optionally substituted monoalkylamino group or dialkylamino group, and any two or more of R₅, R₆, R₇, and Y may together form a ring, and R₆, R₇, X₂, and X₃ may be the same or different when R₆, R₇, X₂, and X₃ are plural.
[4] The paste-form two-pack type dental curable composition according to [3], wherein the nitrogen atom-containing ligand (D) is at least one compound selected from the group consisting of a compound represented by general formula (1), and a compound represented by general formula (2).
[5] The paste-form two-pack type dental curable composition according to any one of [1] to [3], wherein the nitrogen atom-containing ligand (D) is at least one compound selected from the group consisting of N,N,N',N'-tetraethylethylenediamine, N, N, N, N', N", N"-pentamethyldiethylenetriamine, N, N, N', N'-tetramethylpropylenediamine, N,N-bis(2-dimethylaminoethyl)-N,N'-dimethylethylenediamine, 1,4,8,11-tetramethyl-1,4,8,11-tetraazacyclotetradecane, tris[2-(dimethylamino)ethyl]amine, and 2,6-bis(1-pyrazole)-pyridine.
[6] The paste-form two-pack type dental curable composition according to any one of [1] to [3], wherein the nitrogen atom-containing ligand (D) is at least one compound selected from the group consisting of N,N,N,N',N",N"-pentamethyldiethylenetriamine, N, N, N', N'-tetramethylpropylenediam ine, N,N-bis(2-dimethylaminoethyl)-N,N'-dimethylethylenediamine, and tris[2-(dimethylam ino)ethyl]am ine.
[7] The paste-form two-pack type dental curable composition according to any one of [1] to [6], wherein the ratio of the acidic group-containing polymerizable monomer (A) and the nitrogen atom-containing ligand (D) in the first agent is 40:1 to 2:1 (mass ratio).
[8] The paste-form two-pack type dental curable composition according to any one of [1] to [7], which is a self-adhesive composition.

### Advantageous Effects of Invention

According to the present invention, a paste-form two-pack type dental curable composition can be provided that has an adequate working time range and superior bond strength to dentin, and that shows small variation in working time, small decline in bond strength to dentin, and low risk of paste solidification over extended storage.

### DESCRIPTION OF EMBODIMENTS

A paste-form two-pack type dental curable composition of the present invention (hereinafter, also referred to simply as "dental curable composition") comprises:
a first agent that comprises an acidic group-containing polymerizable monomer (A), an acidic group-free polymerizable monomer (B), an organic peroxide (C), a nitrogen atom-containing ligand (D), and a filler (E); and
a second agent that comprises an acidic group-free polymerizable monomer (B), a thiourea compound (F), and a filler (E).

The first agent in a dental curable composition of the present invention is described first. The first agent comprises an acidic group-containing polymerizable monomer (A), an acidic group-free polymerizable monomer (B), an organic peroxide (C), a nitrogen atom-containing ligand (D), and a filler (E).

The acidic group-containing polymerizable monomer (A) is an essential component for a dental curable composition of the present invention to exhibit adhesive properties. The acidic group-containing polymerizable monomer (A) serves to demineralize tooth structure.

Conceivably, the acidic group-containing polymerizable monomer (A) contributes to the extended stabilization of organic peroxide (C) by forming a salt with the nitrogen atom-containing ligand (D), reducing the risk of paste solidification.

The acidic group-containing polymerizable monomer (A) is a polymerizable monomer having at least one acidic group such as a phosphoric acid group, a phosphonic acid group, a pyrophosphoric acid group, a thiophosphoric acid group, a carboxylic acid group, and a sulfonic acid group, and at least one polymerizable group such as an acryloyl group, a methacryloyl group, an acrylamide group, and a methacrylamide group.

In view of adhesive properties to tooth structure, the acidic group-containing polymerizable monomer (A) is preferably a monofunctional polymerizable monomer having any one of an acryloyl group, a methacryloyl group, an acrylamide group, and a methacrylamide group as a polymerizable group. Specific examples of the polymerizable monomer (A) having an acidic group are as follows.

Examples of the phosphoric acid group-containing polymerizable monomer include:
phosphoric acid group-containing monofunctional (meth)acrylate compounds such as 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyeicosyl dihydrogen phosphate, 2-(meth)acryloyloxyethylphenyl hydrogen phosphate, 2-(meth)acryloyloxyethyl-2-bromoethyl hydrogen phosphate, 2-(meth)acryloyloxyethyl-(4-methoxyphenyl)hydrogen phosphate, 2-(meth)acryloyloxypropyl-(4-methoxyphenyl)hydrogen phosphate, and acid chlorides, alkali metal salts, ammonium salts, and amine salts of these; and
phosphoric acid group-containing bifunctional (meth)acrylate compounds such as bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, and acid chlorides, alkali metal salts, ammonium salts, and amine salts of these.

Examples of the phosphonic acid group-containing polymerizable monomer include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, 10-(meth)acryloyloxydecylphosphonoacetate, and acid chlorides, alkali metal salts, ammonium salts, and amine salts of these.

Examples of the pyrophosphoric acid group-containing polymerizable monomer include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, ammonium salts, and amine salts of these.

Examples of the thiophosphoric acid group-containing polymerizable monomer include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyeicosyl dihydrogen thiophosphate, and acid chlorides, alkali metal salts, ammonium salts, and amine salts of these.

Examples of the carboxylic acid group-containing polymerizable monomer include:
(meth)acrylic acid, 4-[2-[(meth)acryloyloxy]ethoxycarbonyl]phthalic acid, 4-(meth)acryloyloxyethyl trimellitic acid, 4-(meth)acryloyloxybutyloxycarbonyl phthalic acid, 4-(meth)acryloyloxyhexyloxycarbonyl phthalic acid, 4-(meth)acryloyloxyoctyloxycarbonyl phthalic acid, 4-(meth)acryloyloxydecyloxycarbonyl phthalic acid, and acid anhydrides of these; and
5-(meth)acryloylaminopentylcarboxylic acid, 6-(meth)acryloyloxyhexane-1,1-dicarboxylic acid, 8-(meth)acryloyloxyoctane-1,1-dicarboxylic acid, 10-(meth)acryloyloxydecane-1,1-dicarboxylic acid, 11-(meth)acryloyloxyundecane-1,1-dicarboxylic acid, and acid chlorides, alkali metal salts, ammonium salts, and amine salts of these.

Examples of the sulfonic acid group-containing polymerizable monomer include 2-(meth)acrylamide-2-methylpropanesulfonic acid, 2-sulfoethyl (meth)acrylate, and acid chlorides, alkali metal salts, ammonium salts, and amine salts of these.

Preferred among these acidic group-containing polymerizable monomers (A) are phosphoric acid group-containing polymerizable monomers, pyrophosphoric acid group-containing polymerizable monomers, and carboxylic acid group-containing polymerizable monomers, more preferably phosphoric acid group-containing polymerizable monomers and carboxylic acid group-containing polymerizable monomers because these polymerizable monomers exhibit even superior adhesion to tooth structure.

Even more preferred are phosphoric acid group-containing (meth)acrylate monofunctional polymerizable monomers or carboxylic acid group-containing (meth)acrylate polymerizable monomers having a C₆ to C₂₀ alkyl group or C₆ to C₂₀ alkylene group as the backbone within the molecule, more preferably phosphoric acid group-containing (meth)acrylate monofunctional polymerizable monomers having a C₈ to C₁₂ alkylene group as the backbone within the molecule.

Particularly preferred are 10-methacryloyloxydecyl dihydrogen phosphate, 4-(meth)acryloyloxyethyl trimellitic acid, and 4-(meth)acryloyloxyethyl trimellitic acid anhydride, most preferably 10-methacryloyloxydecyl dihydrogen phosphate.

The acidic group-containing polymerizable monomer (A) may be incorporated alone, or two or more thereof may be incorporated in combination.

The content of acidic group-containing polymerizable monomer (A) is not particularly limited, as long as the present invention can exhibit its effects. However, in view of even superior adhesive properties, the content of acidic group-containing polymerizable monomer (A) ranges preferably from 1 to 50 parts by mass, more preferably from 2 to 25 parts by mass, even more preferably from 2 to 15 parts by mass in total 100 parts by mass of polymerizable monomer components in a dental curable composition of the present invention.

In this specification, the phrase "total 100 parts by mass of polymerizable monomer components in a dental curable composition" means the content of when the total amount of the polymerizable monomers contained in the first and second agents is converted to 100 parts by mass.

In this specification, "total amount of dental curable composition" means the total amount of the components contained in the first and second agents.

The acidic group-free polymerizable monomer (B) is a polymerizable monomer that undergoes polymerization as a result of a radical polymerization reaction driven by a polymerization initiator system. The polymerizable monomers constituting the acidic group-free polymerizable monomer (B) in the present invention are not limited to one kind of polymerizable monomer, and may be two or more kinds of polymerizable monomers.

Preferred examples of the acidic group-free polymerizable monomer (B) include the hydrophilic polymerizable monomer (B-1) and hydrophobic polymerizable monomer (B-2) below.

Hydrophilic polymerizable monomer (B-1) means a polymerizable monomer having a solubility of 10 mass% or more in water at 25°C. Preferably, the hydrophilic polymerizable monomer (B-1) is one having a solubility of 30 mass% or more in water at 25°C, more preferably one that can dissolve in water in any proportion at 25°C.

The hydrophilic polymerizable monomer (B-1) promotes penetration of the components of the dental curable composition into tooth structure. The hydrophilic polymerizable monomer (B-1) itself also penetrates into tooth structure, and adheres to the organic component (collagen) in the tooth structure.

Examples of the hydrophilic polymerizable monomer (B-1) include monofunctional (meth)acrylic acid ester polymerizable monomers such as 2-hydroxyethyl acrylate, 2-hydroxyethyl methacrylate (hereinafter, also referred to by the abbreviation "HEMA"), 3-hydroxypropyl (meth)acrylate, 2-hydroxypropyl (meth)acrylate, 1,3-dihydroxypropyl (meth)acrylate, 2,3-dihydroxypropyl (meth)acrylate, and 2-((meth)acryloyloxy)ethyltrimethylammonium chloride; and bifunctional (meth)acrylic acid ester polymerizable monomers such as polyethylene glycol di(meth)acrylate (with nine or more oxyethylene groups). 2-Hydroxyethyl (meth)acrylate is preferred in view of adhesive properties to tooth structure.

In this specification, "(meth)acryl" means acryl and methacryl, and the same applies to similar expressions, such as "(meth)acryloyl" and "(meth)acrylate".

Hydrophobic polymerizable monomer (B-2) means a crosslinkable polymerizable monomer having a solubility of less than 10 mass% in water at 25°C.

Examples of the hydrophobic polymerizable monomer (B-2) include aromatic bifunctional polymerizable monomers, aliphatic bifunctional polymerizable monomers, and tri- and higher-functional polymerizable monomers. The hydrophobic polymerizable monomer (B-2) improves properties such as the mechanical strength of cured products of the dental curable composition, and ease of handling.

Examples of the aromatic bifunctional polymerizable monomers include aromatic di(meth)acrylates. Specific examples of the aromatic bifunctional polymerizable monomers include 2,2-bis((meth)acryloyloxyphenyl)propane, 2,2-bis[4-(3-acryloyloxy-2-hydroxypropoxy)phenyl]propane, 2,2-bis[4-(2-hydroxy-3-methacryloyloxypropoxy)phenyl]propane (hereinafter, also referred to by the abbreviation "Bis-GMA"), 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl)pyromellitate. Preferred among these are 2,2-bis[4-(3-methacryloyloxy-2-hydroxypropoxy)phenyl]propane, and 2,2-bis(4-methacryloyloxypolyethoxyphenyl)propane (with an average of 2.6 moles of ethoxy group added; hereinafter, also referred to by the abbreviation "D2.6E").

Examples of the aliphatic bifunctional polymerizable monomers include:
bifunctional (meth)acrylic acid ester polymerizable monomers such as erythritol di(meth)acrylate, sorbitol di(meth)acrylate, mannitol di(meth)acrylate, pentaerythritol di(meth)acrylate, dipentaerythritol di(meth)acrylate, glycerol di(meth)acrylate, ethylene glycol di(meth)acrylate, diethylene glycol di(meth)acrylate, triethylene glycol diacrylate, triethylene glycol dimethacrylate (hereinafter, also referred to by the abbreviation "TEGDMA"), propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 2,2,4-trimethylhexamethylenebis(2-carbamoyloxyethyl)dimethacrylate, and 1,2-bis(3-methacryloyloxy-2-hydroxypropyloxy)ethane; and
(meth)acrylamide polymerizable monomers such as N-methacryloyloxyethylacrylamide, N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide.

Preferred among these are glycerol dimethacrylate, triethylene glycol di(meth)acrylate, neopentyl glycol dimethacrylate, 2,2,4-trimethylhexamethylenebis(2-carbamoyloxyethyl)dimethacrylate, and 1,2-bis(3-methacryloyloxy-2-hydroxypropyloxy)ethane.

Examples of the tri- and higher-functional polymerizable monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N-(2,2,4-trimethylhexamethylene)bis[2-(aminocarboxy)propane-1,3-diol]tetramethacryl ate, and 1,7-diacryloyloxy-2,2,6,6-tetraacryloyloxymethyl-4-oxaheptane.

In view of the mechanical strength and polymerization curability of the cured product, preferred for use among these hydrophobic polymerizable monomers (B-2) are Bis-GMA, D2.6E, and TEGDMA.

The acidic group-free polymerizable monomer (B) (hydrophilic polymerizable monomer (B-1), hydrophobic polymerizable monomer (B-2)) may be incorporated alone, or two or more thereof may be incorporated in combination.

The content of acidic group-free polymerizable monomer (B) is not particularly limited, as long as the present invention can exhibit its effects. However, in view of providing a dental curable composition having high penetrability into the tooth structure and superior adhesive properties, as well as providing a cured product having a sufficient mechanical strength, the content of acidic group-free polymerizable monomer (B) ranges preferably from 50 to 99 parts by mass, more preferably from 60 to 98 parts by mass, even more preferably from 70 to 95 parts by mass in total 100 parts by mass of polymerizable monomer components in a dental curable composition of the present invention.

By incorporating the organic peroxide (C) and thiourea compound (F) as a polymerization initiator system, and combining these with components such as the nitrogen atom-containing ligand (D) within the first and second agents, a dental curable composition can be obtained that, when used by mixing the first agent and second agent, has an adequate working time range and superior bond strength to dentin, and that shows small variation in working time, and small decline in bond strength to dentin over extended storage.

For example, concerning the nitrogen atom-containing ligand (D), while the combination of organic peroxide (C) and thiourea compound (F) becomes dominant in terms of curability in the overall system upon mixing and using the dental curable composition, the curability of the polymerization initiator system is also influenced by the system's overall acidity. As such, the nitrogen atom-containing ligand (D), which forms a salt with the acidic group-containing polymerizable monomer (A), also influences the working time and adhesive properties over extended storage within the entire system.

As described above, each component acts integrally to reduce the risk of paste solidification even after extended storage, while also achieving small variation in working time, and small decline in bond strength to dentin.

Examples of the organic peroxide (C) include diacyl peroxides, peroxyesters, dialkyl peroxides, peroxyketals, ketone peroxides, and hydroperoxides.

Specific examples of the diacyl peroxides include benzoyl peroxide, 2,4-dichlorobenzoyl peroxide, and m-toluoyl peroxide.

Specific examples of the peroxyesters include t-butyl peroxybenzoate, bis(t-butylperoxy)isophthalate, 2,5-dimethyl-2,5-bis(benzoylperoxy)hexane, t-butyl peroxy-2-ethylhexanoate, and t-butyl peroxyisopropyl carbonate.

Specific examples of the dialkyl peroxides include dicumyl peroxide, di-t-butyl peroxide, and lauroyl peroxide.

Specific examples of the peroxyketals include
1,1-bis(t-butylperoxy)3,3,5-trimethylcyclohexane, 1,1-bis(t-butylperoxy)cyclohexane, and 1,1-bis(t-hexylperoxy)cyclohexane.

Specific examples of the ketone peroxides include methyl ethyl ketone peroxide, cyclohexanone peroxide, and methyl acetoacetate peroxide.

Specific examples of the hydroperoxides include t-butyl hydroperoxide, cumene hydroperoxide, p-diisopropylbenzene hydroperoxide, and 1,1,3,3-tetramethylbutyl hydroperoxide.

Particularly preferred as organic peroxide (C) are hydroperoxides and peroxyesters. Preferred for use as hydroperoxides are t-butyl hydroperoxide, cumene hydroperoxide, and 1,1,3,3-tetramethylbutyl hydroperoxide. Preferred for use as peroxyesters is t-butyl peroxybenzoate.

The organic peroxide (C) may be incorporated alone, or two or more thereof may be incorporated in combination.

In view of curability, the mechanical strength of the cured product, and adhesive properties to tooth structure, the content of organic peroxide (C) ranges preferably from 0.01 to 10 parts by mass, more preferably from 0.1 to 5 parts by mass, even more preferably from 0.5 to 3 parts by mass with respect to total 100 parts by mass of polymerizable monomer components in the dental curable composition.

In a dental curable composition of the present invention, the nitrogen atom-containing ligand (D) contributes to reducing the risk of solidification of the first agent, and stabilizing the working time over extended storage.

Preferably, the nitrogen atom-containing ligand (D) comprises a ligand having two or more nitrogen atoms within the molecule. More preferably, the nitrogen atom-containing ligand (D) is a polydentate ligand having two or more nitrogen atoms within the molecule.

More preferably, the nitrogen atom-containing ligand (D) is at least one compound selected from the group consisting of a compound represented by general formula (1), a compound represented by general formula (2), and a polydentate ligand (3) containing a nitrogen-containing heterocyclic ring. Particularly, by incorporating nitrogen atom-containing ligands having specific structures, such as polydentate ligands having two or more nitrogen atoms, the nitrogen atom-containing ligand (D) can enhance its contribution in reducing the risk of solidification and stabilizing the working time.

The nitrogen atom-containing ligand (D) may be a compound with no sulfur atom.

Among the nitrogen atom-containing ligands (D), the increased effectiveness of polydentate ligands can be explained as follows.

The nitrogen atom-containing ligand (D) is presumed to contribute to the prolonged stabilization of organic peroxide (C) by forming a salt with the acidic group-containing polymerizable monomer (A). While the movement of ligand (D) molecules containing a nitrogen atom is restricted within the first agent paste, a greater number of nitrogen atoms per molecule enhances the likelihood of contact with the acidic group-containing polymerizable monomer (A), making the reaction more advantageous from a physicochemical standpoint.

R₁R₂N-X₁-NR₃R₄ (1),

wherein R₁ to R₄ each independently represent an optionally substituted alkyl group, and X₁ represents an optionally substituted divalent aliphatic group. 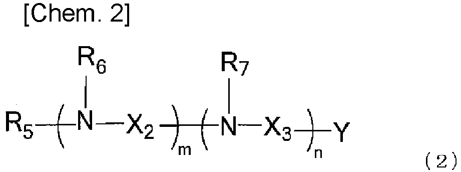 wherein R₅, R₆, and R₇ each independently represent an optionally substituted alkyl group, X₂ and X₃ each independently represent an optionally substituted divalent aliphatic group that may contain an oxygen atom and/or a nitrogen atom, m and n each independently represent an integer of 1 or more, Y represents an optionally substituted monoalkylamino group or dialkylamino group, and any two or more of R₅, R₆, R₇, and Y may together form a ring, and R₆, R₇, X₂, and X₃ may be the same or different when R₆, R₇, X₂, and X₃ are plural.

The optionally substituted alkyl groups represented by R₁ to R₄ may be linear or branched. The number of carbon atoms in the alkyl groups represented by R₁ to R₄ is not particularly limited, and is preferably 1 to 12, more preferably 1 to 6, even more preferably 1 to 4, particularly preferably 1 to 3.

Examples of the alkyl groups represented by R₁ to R₄ include methyl groups, ethyl groups, n-propyl groups, isopropyl groups, n-butyl groups, isobutyl groups, sec-butyl groups, tert-butyl groups, n-pentyl groups, isopentyl groups, sec-pentyl groups, neopentyl groups, n-hexyl groups, isohexyl groups, n-heptyl groups, n-octyl groups, n-nonyl groups, n-decyl groups, n-undecyl groups, and n-dodecyl groups. The alkyl groups represented by R₁ to R₄ may be unsubstituted.

Examples of the substituents on the alkyl groups represented by R₁ to R₄ include halogen atoms (a fluorine atom, a chlorine atom, a bromine atom, an iodine atom), hydroxyl groups, alkoxy groups having 1 to 6 carbon atoms, dialkylamino groups having 1 to 6 carbon atoms in each alkyl group, and amino groups.

R₁ to R₄ may be the same or different. For example, R₁ to R₄ may partly represent the same alkyl groups (for example, R₁ and R₃ may be the same alkyl groups).

The optionally substituted divalent aliphatic group represented by X₁ may be linear or branched. The divalent aliphatic group has preferably 1 to 20 carbon atoms, more preferably 1 to 16 carbon atoms, even more preferably 1 to 12 carbon atoms, particularly preferably 1 to 8 carbon atoms.

Examples of the divalent aliphatic group represented by X₁ include alkylene groups, alkenylene groups, and alkynylene groups, of which alkylene groups are preferred.

Examples of the alkylene groups include methylene groups, ethylene groups, propylene groups, butylene groups, methylpropylene groups, dimethylpropylene groups, pentamethylene groups, hexamethylene groups, heptamethylene groups, octamethylene groups, nonamethylene groups, decamethylene groups, undecamethylene groups, and dodecamethylene groups.

Examples of the substituents on the divalent aliphatic group represented by X₁ include the same substituents as those of the alkyl groups represented by R₁ to R₄.

A certain preferred embodiment is, for example, a paste-form two-pack type dental curable composition in which the nitrogen atom-containing ligand (D) is a compound represented by general formula (1), wherein, in general formula (1), R₁ to R₄ each independently represent a linear or branched C1 to C6 alkyl group that may have a substituent, and X₁ is a linear or branched C1 to C8 alkylene group that may have a substituent.

The optionally substituted alkyl groups represented by R₅, R₆, and R₇ are as described for the optionally substituted alkyl groups represented by R₁ to R₄. The number of carbon atoms in the monoalkylamino group (-NHR^{a} (R^{a} represents an alkyl group)) and dialkylamino group (-NR^{b}R^{c} (R^{b} and R^{c} represent alkyl groups)) represented by Y is not particularly limited, and is preferably 1 to 12, more preferably 1 to 6, even more preferably 1 to 4, particularly preferably 1 to 3.

Examples of the alkyl groups in the monoalkylamino group and dialkylamino group represented by Y include the optionally substituted alkyl groups represented by R₁ to R₄ satisfying the foregoing carbon counts.

The dialkylamino group may be one having the foregoing carbon counts in each alkyl group.

Examples of the optionally substituted monoalkylamino group represented by Y include methylamino groups, ethylamino groups, propylamino groups, isopropylamino groups, butylamino groups, isobutylamino groups, t-butylamino groups, pentylamino groups, and hexylamino groups.

Examples of the optionally substituted dialkylamino group represented by Y include dimethylamino groups, diethylamino groups, dipropylamino groups, diisopropylamino groups, dibutylamino groups, diisobutylamino groups, dipentylamino groups, dihexylamino groups, and ethylmethylamino groups.

The alkyl groups in the monoalkylamino group and dialkylamino group represented by Y may be substituted with a substituent.

Examples of the substituent include the same substituents as those of the alkyl groups represented by R₁ to R₄.

The divalent aliphatic groups represented by X₂ and X₃ are the same as the optionally substituted divalent aliphatic group represented by X₁, except that the divalent aliphatic groups represented by X₂ and X₃ may contain an oxygen atom and/or a nitrogen atom. X₂ and X₃ may be the same or different.

The number of oxygen atoms and nitrogen atoms contained in the divalent aliphatic groups represented by X₂ and X₃ is one or more, and may be two or more. When the divalent aliphatic groups represented by X₂ and X₃ contain a nitrogen atom, the divalent aliphatic groups represented by X₂ and X₃ may contain -NH-, or -NR^{d} (R^{d} represents a C1 to C6 alkyl group)-.

The symbols m and n each independently represent an integer of 1 or more, preferably an integer of 1 to 8, more preferably an integer of 1 to 6, even more preferably an integer of 1 to 5, particularly preferably an integer of 1 to 3.

The symbols m and n may be the same or different. X₂ and X₃ may be the same or different from each other when m and n are 2 or more, and X₂ and X₃ are plural.

Any two or more of R₅, R₆, R₇, and Y may together form a ring. For example, R₅, R₆, or R₇ may form a ring with Y Alternatively, R₅ and Y, and R₆ and R₇ may separately form a ring, creating a compound with two rings. Furthermore, the nitrogen atom in the amino group represented by Y may form a ring with R₅. The ring may contain an oxygen atom and/or a nitrogen atom.

In certain embodiments, compounds represented by general formula (2) may be compounds having a bicyclic ring. For example, in other embodiments, compounds represented by general formula (2) may be compounds having a bicyclic ring, with a ring formed by Y and R₅, and another ring formed by R₆ and Y or R₇ and Y

In a certain preferred embodiment, R₅, R₆, and R₇ are linear or branched C1 to C6 alkyl groups that may have a substituent.

Another certain preferred embodiment is, for example, a paste-form two-pack type dental curable composition in which the nitrogen atom-containing ligand (D) is at least one compound selected from the group consisting of a compound represented by general formula (1), and a compound represented by general formula (2).

Yet another certain preferred embodiment is, for example, a paste-form two-pack type dental curable composition in which the nitrogen atom-containing ligand (D) is a compound represented by general formula (2).

Still another certain preferred embodiment is, for example, a paste-form two-pack type dental curable composition in which the nitrogen atom-containing ligand (D) is a compound represented by general formula (2), wherein, in the compound represented by general formula (2), R₅, R₆, and R₇ represent linear or branched C1 to C6 alkyl groups that may have a substituent, the divalent aliphatic groups represented by X₂ and X₃ represent C1 to C8 alkylene groups containing no oxygen atom and no nitrogen atom, m and n each independently represent an integer of 1 or more, Y represents an optionally substituted monoalkylamino group or dialkylamino group, Y and R₅ may together form a ring, and R₆, R₇, X₂, and X₃ may be the same or different when R₆, R₇, X₂, and X₃ are plural.

Another certain preferred embodiment is, for example, a paste-form two-pack type dental curable composition in which the nitrogen atom-containing ligand (D) is a compound represented by general formula (2), wherein, in general formula (2), m is 1 and n is 2, creating a compound containing four nitrogen atoms as a whole.

The polydentate ligand (3) containing a nitrogen-containing heterocyclic ring is a bidentate or higher dentate ligand compound that comprises a heterocyclic ring containing a five-membered or six-membered ring having a nitrogen atom, and has two or more nitrogen atoms within the molecule. The polydentate ligand (3) has two or more nitrogen atoms within the molecule, and may have three or more nitrogen atoms within the molecule. The polydentate ligand (3) may contain one heterocyclic ring, or two or more heterocyclic rings.

Examples of the nitrogen-containing heterocyclic ring include nitrogen-containing five-membered rings such as a pyrrole ring, a pyrazole ring, and an imidazole ring; and a nitrogen-containing six-membered rings such as a pyridine ring, a pyrazine ring, a pyridazine ring, a piperazine ring, a pyrimidine ring, and a triazine ring.

The nitrogen-containing heterocyclic ring may be a fused ring formed by the nitrogen atom-containing five-membered or six-membered ring with other rings (for example, an aromatic ring), or a fused ring formed by nitrogen atom-containing five-membered or six-membered rings. Examples of the fused rings formed by the nitrogen atom-containing five-membered or six-membered ring with an aromatic ring include a quinoline ring, an isoquinoline ring, an indole ring, a benzoimidazole ring, and a benzotriazole ring.

The polydentate ligand (3) comprises a heterocyclic ring containing a nitrogen atom-containing five-membered or six-membered ring. An example is a ligand compound that comprises a fused ring, such as an indole ring, a benzoimidazole ring, or a benzotriazole ring, and a heterocyclic ring containing a nitrogen atom-containing five-membered or six-membered ring. Concerning denticity, the polydentate ligand (3) is a bidentate or higher dentate ligand compound, and may be tridentate or tetradentate, for example.

Examples of compounds represented by the general formula (1) include two-coordinate polydentate amine compounds such as N,N,N',N'-tetramethylethylenediamine (hereinafter, also referred to by the abbreviation "TMEDA"), N,N,N',N'-tetramethylpropylenediamine (hereinafter, also referred to by the abbreviation "TMPDA"), N,N,N',N'-tetramethyl-1,4-diaminobutane, N,N,N',N'-tetraethylethylenediamine (hereinafter, also referred to by the abbreviation "TEEDA"), and N,N,N',N'-tetrakis(2-hydroxyethyl)ethylenediamine.

Examples of compounds represented by the general formula (2) include polydentate amine compounds, for example, compounds having a cyclic ring, such as 1,4,8,11-tetramethyl-1,4,8,11-tetraazacyclotetradecane (hereinafter, also referred to by the abbreviation "TMTACTD"), compounds having a bicyclic ring such as 4,11-dimethyl-1,4,8,11-tetraazabicyclohexadecane, and compounds with no rings, such as 2,5,9,12-tetramethyl-2,5,9,12-tetraazatetradecane, 2,6,9,13-tetramethyl-2,6,9,13-tetraazatetradecane, 2,5,8,12-tetramethyl-2,5,8,12-tetraazatetradecane, N,N,N,N',N",N"-pentamethyldiethylenetriamine (hereinafter, also referred to by the abbreviation "PMDETA"), hexamethyltris(2-aminoethyl)amine, N,N-bis(2-dimethylaminoethyl)-N,N'-dimethylethylenediamine (hereinafter, also referred to by the abbreviation "HMTETA"), and tris[2-(dimethylamino)ethyl]amine (hereinafter, also referred to by the abbreviation "Me₆TREN").

Examples of the polydentate ligand (3) containing a nitrogen-containing heterocyclic ring include polydentate ligands having one nitrogen-containing heterocyclic ring, such as N-(n-propyl)pyridylmethaneimine, and N-(n-octyl)pyridylmethaneimine, and polydentate ligands having two or more nitrogen-containing heterocyclic rings, such as 2,2-bipyridine, 4,4'-di-(5-nonyl)-2,2'-bipyridine, N-propyl-N,N-di(2-pyridylmethyl)amine, N',N"-dimethyl-N',N"-bis((pyridin-2-yl)methyl)ethane-1,2-diamine, 2,6-bis(1-pyrazole)-pyridine (hereinafter, also referred to by the abbreviation "DPP"), 2-(2-pyridyl)benzoimidazole, tris[(2-pyridyl)methyl]amine, 3,6-di(2-pyridyl)-1,2,4,5-tetrazine, N,N,N',N'-tetrakis(2-pyridylmethyl)ethylenediamine, and 2,4,6-tri(2-pyridyl)-1,3,5-triazine.

A certain preferred embodiment is, for example, a paste-form two-pack type dental curable composition in which the nitrogen atom-containing ligand (D) is at least one compound selected from the group consisting of N,N,N',N'-tetraethylethylenediamine, N,N,N,N',N",N"-pentamethyldiethylenetriamine, N,N,N',N'-tetramethylpropylenediamine, N,N-bis(2-dimethylaminoethyl)-N,N'-dimethylethylenediamine, 1,4,8,11-tetramethyl-1,4,8,11-tetraazacyclotetradecane, tris[2-(dimethylamino)ethyl]amine, and 2,6-bis(1-pyrazole)-pyridine.

Another certain preferred embodiment is, for example, a paste-form two-pack type dental curable composition in which the nitrogen atom-containing ligand (D) is at least one compound selected from the group consisting of N,N,N,N',N",N"-pentamethyldiethylenetriamine, N,N,N',N'-tetramethylpropylenediamine, N,N-bis(2-dimethylaminoethyl)-N,N'-dimethylethylenediamine, and tris[2-(dimethylamino)ethyl]amine.

Among these nitrogen atom-containing ligands (D), the amine compounds represented by general formula (1) and the amine compounds represented by general formula (2) are preferred in view of their ability to provide even smaller variation in working time and greater stabilization of working time over extended storage periods, and achieve an even smaller decline in bond strength to dentin over extended storage.

The nitrogen atom-containing ligand (D) may be incorporated alone, or two or more thereof may be incorporated in combination.

In view of curability, the mechanical strength of the cured product, adhesive properties to tooth structure, and stabilization of working time, the content of nitrogen atom-containing ligand (D) ranges preferably from 0.05 to 10 parts by mass, more preferably from 0.1 to 8 parts by mass, even more preferably from 0.5 to 5 parts by mass with respect to total 100 parts by mass of polymerizable monomer components in the dental curable composition.

In view of curability, the mechanical strength of the cured product, adhesive properties to tooth structure, and stabilization of working time, the ratio (mass ratio) of the acidic group-containing polymerizable monomer (A) and nitrogen atom-containing ligand (D) contained in the first agent is preferably 40:1 to 2:1, more preferably 30:1 to 4:1, even more preferably 20:1 to 6:1.

The first agent in a dental curable composition of the present invention is paste form, and comprises a filler (E) to provide sufficient workability to the composition, and sufficient X-ray opacity and mechanical strength to the cured product.

The filler (E) may be any type of filler, provided that it does not hinder the effectiveness of the present invention. Examples include inorganic fillers, organic fillers, and composite fillers of inorganic fillers and organic fillers.

The filler (E) may be incorporated alone, or two or more thereof may be incorporated in combination.

The filler (E) has an average particle diameter of preferably 0.001 to 10 µm, more preferably 0.001 to 5 µm.

Examples of the materials of the inorganic fillers include quartz, silica, alumina, silica-titania, silica-titania-barium oxide, silica-zirconia, silica-alumina, lanthanum glass, borosilicate glass, soda glass, barium glass, strontium glass, glass-ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, strontium calcium fluoroaluminosilicate glass, ytterbium oxide, and silica-coated ytterbium fluoride.

In view of ease of handling, preferred for use as inorganic fillers are particulate silica having an average particle diameter of 0.001 to 0.1 µm.

A certain preferred embodiment is, for example, a separate-pack type dental curable composition in which the filler (E) in the first agent comprises an inorganic filler having an average particle diameter of 0.001 µm or more and 0.1 µm or less, and an inorganic filler having an average particle diameter of more than 0.1 µm and 10 µm or less.

The inorganic fillers may be commercially available products. Examples of such commercially available products include Aerosil^{®} OX50, Aerosil^{®} 50, Aerosil^{®} 200, Aerosil^{®} 380, Aerosil^{®} R972, Aerosil^{®} 130, and AEROXIDE^{®} Alu C (all manufactured by Nippon Aerosil Co., Ltd. under these trade names).

In the present invention, the average particle diameter of inorganic filler means the average particle diameter before surface treatment when the inorganic filler is surface treated as will be described later.

Examples of the organic fillers include polymethyl methacrylate, polyethyl methacrylate, polymers of polyfunctional methacrylate, polyamides, polystyrene, polyvinyl chloride, chloroprene rubber, nitrile rubber, and styrene-butadiene rubber.

Examples of the composite fillers of inorganic fillers and organic fillers include those comprising inorganic fillers dispersed in organic fillers, and inorganic/organic composite fillers comprising various types of polymers coating the inorganic filler.

In view of the transparency of the dental curable composition and the mechanical strength of the cured product obtained, preferred among these fillers (E) are inorganic fillers such as quartz, silica, silica-zirconia, barium glass, ytterbium oxide, and silica-coated ytterbium fluoride, more preferably quartz, silica, silica-zirconia, barium glass, and silica-coated ytterbium fluoride.

In view of reduced interaction with the acidic group-containing polymerizable monomer (A) and smaller changes in paste properties over prolonged storage, it is even more preferable in the first agent that the filler (E) be a combination of either quartz or silica with silica-coated ytterbium fluoride. In this case, the X-ray contrast properties of the resulting first agent can be adjusted by appropriately adjusting the content of silica-coated ytterbium fluoride.

The filler (E) may be used after a surface treatment with a known surface treatment agent such as a silane coupling agent, in order to improve curability, the mechanical strength of the cured product, and ease of handling. Examples of such surface treatment agents include vinyltrimethoxysilane, vinyltriethoxysilane, vinyltrichlorosilane, vinyltri(β-methoxyethoxy)silane, γ-methacryloyloxypropyltrimethoxysilane, γ-glycidoxypropyltrimethoxysilane, γ-mercaptopropyltrimethoxysilane, and γ-aminopropyltriethoxysilane.

The average particle diameter (average primary particle diameter) can be determined by a laser diffraction scattering method or by electron microscopy of particles. Specifically, a laser diffraction scattering method is more convenient for the measurement of particles that are 0.1 µm or larger, whereas electron microscopy is a more convenient method of particle diameter measurement for ultrafine particles of less than 0.1 µm. Here, a value of 0.1 µm is the reference value, measured by a laser diffraction scattering method.

As an example of a laser diffraction scattering method, the particle size may be measured by volume using a laser diffraction particle size distribution analyzer (SALD-2300, manufactured by Shimadzu Corporation) with a 0.2% sodium hexametaphosphate aqueous solution used as dispersion medium.

A scanning electron microscope (e.g., SU3800 or S-4000 manufactured by Hitachi High-Technologies Corporation) may be used for electron microscopy. In electron microscopy, the particle size can be measured by taking an electron micrograph of particles, and the size of particles (at least 200 particles) observed in a unit field of the captured image may be measured using image-analyzing particle size distribution measurement software (Mac-View, manufactured by Mountech Co., Ltd.). Here, the particle diameter is determined as an arithmetic mean value of the maximum and minimum lengths of particles, and the average primary particle diameter is calculated from the number of particles and the particle diameter.

The content of the filler (E) contained in the first agent is not particularly limited, as long as the present invention can exhibit its effects. However, the content of the filler (E) in the first agent ranges preferably from 50 to 350 parts by mass, more preferably from 80 to 300 parts by mass, even more preferably from 100 to 300 parts by mass, particularly preferably from 100 to 280 parts by mass with respect to 100 parts by mass of polymerizable monomers contained in the first agent of a dental curable composition of the present invention.

In certain embodiments, the content of the filler (E) in the first agent is preferably 50 to 97 mass%, more preferably 55 to 95 mass%, even more preferably 60 to 90 mass% in total 100 mass% of the first agent, though it is not particularly limited as long as the present invention can exhibit its effects.

In view of X-ray contrast properties, it is preferable in certain preferred embodiments that the content of silica-coated ytterbium fluoride, when it is used as a part of the filler (E) in the first agent, fall in the range of preferably 2 to 40 mass%, more preferably 5 to 30 mass%, even more preferably 10 to 25 mass% in total 100 mass% of the first agent.

The following describes the second agent in a dental curable composition of the present invention. The second agent comprises an acidic group-free polymerizable monomer (B), a thiourea compound (F), and a filler (E).

Examples of the acidic group-free polymerizable monomer (B) in the second agent include those exemplified for the acidic group-free polymerizable monomer (B) used for the first agent.

By incorporating the organic peroxide (C) and thiourea compound (F) as a polymerization initiator system, and combining these with components such as the nitrogen atom-containing ligand (D) within the first and second agents, a dental curable composition can be obtained that has an adequate working time range and superior bond strength to dentin, and that shows small variation in working time, and small decline in bond strength to dentin over extended storage.

Examples of the thiourea compound (F) include thiourea, methylthiourea, ethylthiourea, ethylenethiourea, N,N'-dimethylthiourea, N,N'-diethylthiourea, N,N'-di-n-propylthiourea, N,N'-dicyclohexylthiourea, N-acetylthiourea, N-benzoylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 1-(2-pyridyl)-2-thiourea, 4,4-dimethylethylenethiourea, diphenylthiourea, N-(6-methyl-2-pyridyl)-thiourea, 1-(2-(6-ethylpyridyl))-2-thiourea, 1-(2-(6-methoxypyridyl))-2-thiourea, 1-(2-(6-chloropyridyl))-2-thiourea, 1-(2-(5-methylpyridyl))-2-thiourea, 1-(2-(5-ethylpyridyl))-2-thiourea, 1-(2-(5-methoxypyridyl))-2-thiourea, 1-(2-(5-chloropyridyl))-2-thiourea, 1-(2-(5,6-dimethylpyridyl))-2-thiourea, 4-methyl-2-imidazolidinethione, 4,4-dimethyl-2-imidazolidinethione, 4-ethyl-2-imidazolidinethione, and 4,4-diethyl-2-imidazolidinethione.

The thiourea compound (F) may be used alone, or two or more thereof may be used in combination.

In view of curability, the mechanical strength of the cured product, and adhesive properties to tooth structure, the content of thiourea compound (F) ranges preferably from 0.05 to 5 parts by mass, more preferably from 0.1 to 3 parts by mass, even more preferably from 0.2 to 2 parts by mass with respect to total 100 parts by mass of polymerizable monomer components in the dental curable composition.

Examples of the filler (E) in the second agent include those exemplified for the filler (E) used for the first agent.

A certain preferred embodiment is, for example, a separate-pack type dental curable composition in which the filler (E) in the second agent comprises an inorganic filler having an average particle diameter of 0.001 to 0.1 µm, and an inorganic filler having an average particle diameter of more than 0.1 µm and 10 µm or less.

In order to improve curability, the mechanical strength of the cured product, and ease of handling, the filler (E) may be used after a surface treatment with a known surface treatment agent such as a silane coupling agent, as with the case of the first agent. Examples of such surface treatment agents include the same silane coupling agents exemplified for the first agent.

In view of the transparency of the dental curable composition and the mechanical strength of the cured product obtained, preferred as the filler (E) in the second agent are quartz, silica, silica-zirconia, barium glass, and silica-coated ytterbium fluoride.

In view of X-ray contrast properties, the filler (E) in the second agent is more preferably at least one selected from the group consisting of quartz, silica, and silica-zirconia, in combination with silica-coated ytterbium fluoride. Barium glass represents another more preferred example of the filler (E) in the second agent from the perspective of X-ray contrast properties.

The content of the filler (E) contained in the second agent is not particularly limited, as long as the present invention can exhibit its effects. However, the content of the filler (E) contained in the second agent ranges preferably from 50 to 350 parts by mass, more preferably from 80 to 330 parts by mass, even more preferably from 100 to 310 parts by mass, particularly from 100 to 300 parts by mass with respect to 100 parts by mass of polymerizable monomers contained in the second agent of a dental curable composition of the present invention.

In certain embodiments, the content of the filler (E) contained in the second agent is preferably 50 to 97 mass%, more preferably 55 to 95 mass%, even more preferably 60 to 90 mass% with respect to total 100 mass% of the second agent, though it is not particularly limited as long as the present invention can exhibit its effects.

The following describes the optional components in a dental curable composition of the present invention.

A dental curable composition of the present invention comprises a redox-type polymerization initiator, and may additionally comprise polymerization accelerators, for example, such as sulfinic acid and salts thereof, transition metal compounds, ascorbic acid compounds, sulfites, bisulfites, borate compounds, and barbituric acid and its derivatives. Individually, these may be used alone, or two or more thereof may be used in combination.

Examples of the sulfinic acid and salts thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Preferred among these are sodium benzenesulfinate, sodium p-toluenesulfinate, 2,4,6-triisopropylbenzenesulfinic acid, and sodium 2,4,6-triisopropylbenzenesulfinate.

In view of storage stability, it is preferable that the sulfinic acid and salts thereof be incorporated in the second agent. The sulfinic acid and salts thereof may be incorporated alone, or two or more thereof may be incorporated in combination.

In view of curability, the mechanical strength of the cured product, and adhesive properties to tooth structure, the content of the sulfinic acid and salts thereof ranges preferably from 0.01 to 5 parts by mass, more preferably from 0.1 to 4 parts by mass, even more preferably from 0.3 to 3 parts by mass with respect to total 100 parts by mass of polymerizable monomer components in the dental curable composition.

Preferred for use as the transition metal compounds are copper compounds and vanadium compounds.

Examples of the copper compounds include copper(II) carboxylates, copper(II) β-diketones, copper(II) β-ketoesters, copper alkoxides, copper dithiocarbamates, and salts of copper and inorganic acids.

Examples of the copper(II) carboxylates include copper(II) citrate, copper(II) acetate, copper(II) phthalate, copper(II) tartarate, copper(II) oleate, copper(II)octylate, copper(II) octenate, copper(II) naphthenate, copper(II) methacrylate, and copper(II) 4-cyclohexylbutyrate.

Examples of the copper(II) β-diketones include copper(II) acetylacetonate, copper(II) trifluoroacetylacetonate, copper(II) hexafluoroacetylacetonate, copper(II) 2,2,6,6-tetramethyl-3,5-heptanedionate, and copper(II) benzoylacetonate.

Examples of the copper(II) β-ketoesters include copper(II) ethylacetoacetate.

Examples of the copper alkoxides include copper(II) methoxide, copper(II) ethoxide, copper(II) isopropoxide, copper(II) 2-(2-butoxyethoxy)ethoxide, and copper(II) 2-(2-methoxyethoxy)ethoxide.

Examples of the copper dithiocarbamates include copper(II) dimethyldithiocarbamate.

Examples of the salts of copper and inorganic acids include copper(II) nitrate, copper(II) bromide, and copper(II) chloride.

These may be used alone, or two or more thereof may be used in appropriate combination. In view of solubility and reactivity to the polymerizable monomers, preferred are copper(II) carboxylates, copper(II) β-diketones, and copper(II) β-ketoesters, more preferably copper(II) acetate, and copper(II) acetylacetonate.

Preferred as the vanadium compounds are vanadium compounds with a valency of IV and/or V.

Examples of the vanadium compounds with a valency of IV and/or V include vanadium(IV) oxide, vanadyl(IV) acetylacetonate, vanadium(IV) oxide stearate, oxalic acid oxovanadium(IV), vanadyl(IV) sulfate, vanadium naphthenate, vanadium benzoylacetonate, bis(maltolato)oxovanadium(IV), oxobis(1-phenyl-1,3-butanedionate)vanadium(IV), vanadium(V) oxide, vanadium(V) oxytriisopropoxide, sodium metavanadate, and ammonium metavanadate(V). In view of considerations such as adhesive properties, preferred are vanadium(IV) oxide, vanadyl(IV) sulfate, vanadyl(IV) acetylacetonate, and bis(maltolato)oxovanadium(IV), more preferably vanadyl(IV) acetylacetonate and bis(maltolato)oxovanadium(IV).

The vanadium compounds may be used alone, or two or more thereof may be used in combination.

The transition metal compounds may be incorporated alone, or two or more thereof may be incorporated in combination.

In view of storage stability, it is preferable that the transition metal compounds be incorporated in the second agent.

In view of curability, the mechanical strength of the cured product, and adhesive properties to tooth structure, the content of transition metal compound ranges preferably from 0.0001 to 1 part by mass, more preferably from 0.0005 to 0.5 parts by mass, even more preferably from 0.001 to 0.3 parts by mass with respect to total 100 parts by mass of polymerizable monomer components in the dental curable composition.

Examples of the ascorbic acid compounds include salts, esters, and ethers of ascorbic acid. Preferred among these are salts and esters of ascorbic acid.

Examples of salts of ascorbic acid include sodium L-ascorbate, calcium L-ascorbate, potassium ascorbate, and stereoisomers of these (for example, sodium isoascorbate). Preferred among these is sodium L-ascorbate.

Examples of esters of ascorbic acid include those formed by a reaction of carboxylic acid with one or more of the hydroxyl groups of ascorbic acid. Preferred examples of carboxylic acids include fatty acids such as C6 to C30 saturated fatty acids or unsaturated fatty acids, for example, caprylic acid, capric acid, lauric acid, myristic acid, palmitic acid, stearic acid, arachidic acid, behenic acid, lignoceric acid, cerotic acid, myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, and docosahexaenoic acid. The fatty acids have preferably 10 to 28 carbon atoms, more preferably 12 to 26 carbon atoms, even more preferably 14 to 24 carbon atoms. Particularly preferred among these are esters of stearic acid and ascorbic acid, and esters of palmitic acid and ascorbic acid (ascorbyl palmitate).

Examples of ethers of ascorbic acid include ethyl ascorbic ether, and cetyl ascorbic ether.

The ascorbic acid compounds may be incorporated alone, or two or more thereof may be incorporated in combination.

In view of curability, the mechanical strength of the cured product, and adhesive properties to tooth structure, the content of ascorbic acid compound ranges preferably from 0.01 to 5 parts by mass, more preferably from 0.1 to 3 parts by mass, even more preferably from 0.3 to 2 parts by mass with respect to total 100 parts by mass of polymerizable monomer components in the dental curable composition.

In view of storage stability, it is preferable that the ascorbic acid compounds be incorporated in the second agent. The ascorbic acid compounds may be dissolved in a composition of the second agent, or may be dispersed therein in powder form.

When the ascorbic acid compounds are dispersed in powder form, the average particle diameter of the powder is preferably 20 µm or less, more preferably 10 µm or less, even more preferably 5 µm or less, because workability or curability tends to decrease when the average particle diameter is excessively large.

The average particle diameter of a powder of ascorbic acid compounds can be calculated as a volume average particle diameter after an image analysis with image-analyzing particle size distribution measurement software (Mac-View, manufactured by Mountech Co., Ltd.) based on an electron micrograph of at least 100 particles.

The particle shape of when the ascorbic acid compounds are dispersed in powder form is not particularly limited, and the particles may have various shapes, for example, such as spherical, needle-like, plate-like, and crushed shapes. The ascorbic acid compounds can be prepared by known methods such as pulverization, freeze drying, and reprecipitation. In view of the average particle diameter of the resultant powder, preferred are pulverization and freeze drying.

Examples of the sulfites include sodium sulfite, potassium sulfite, calcium sulfite, and ammonium sulfite.

Examples of the bisulfites include sodium bisulfite, and potassium bisulfite.

Examples of the borate compounds include arylborate compounds having 1 to 4 aryl groups per molecule (for example, tetraphenylboron, tetrakis(p-chlorophenyl)boron), and salts of these.

Examples of the barbituric acid and its derivatives include barbituric acid, 5-butylbarbituric acid, 1,3,5-trimethylbarbituric acid, 1-cyclohexyl-5-ethylbarbituric acid, 1-benzyl-5-phenylbarbituric acid, and salts of these.

A dental curable composition of the present invention comprises a redox-type polymerization initiator. However, in order to provide a dual-cure composition also capable of polymerizing upon exposure to light, a known photopolymerization initiator may additionally be incorporated in at least one of the first and second agents, separately from the polymerization initiator system described above.

Examples of the photopolymerization initiator include α-diketones, ketals, thioxanthones, (bis)acylphosphine oxides, and α-aminoacetophenones.

Examples of the α-diketones include dl-camphorquinone (commonly known as CQ), benzyl 2,3-pentanedione.

Examples of the ketals include benzyl dimethyl ketal, and benzyl diethyl ketal.

Examples of the thioxanthones include 2-chlorothioxanthone, and 2,4-diethylthioxanthone.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,3,5,6-tetramethylbenzoyldiphenylphosphine oxide, 2,4,6-trimethylbenzoylphenylphosphine oxide, 2,4,6-trimethylbenzoylmethoxyphenylphosphine oxide, 2,4,6-trimethylbenzoylethoxyphenylphosphine oxide, benzoylbis(2,6-dimethylphenyl)phosphine oxide, the water-soluble acylphosphine oxide compounds disclosed in JP H03-57916 B, and salts of these (for example, sodium salts, potassium salts, ammonium salts).

Examples of bisacylphosphine oxides include bis(2,4,6-trimethylbenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)-2,4,4-trimethylpentylphosphine oxide, bis(2,6-dimethoxybenzoyl)-2,5-dimethylphenylphosphine oxide, dibenzoylphenylphosphine oxide, bis(2,6-dimethoxybenzoyl)phenylphosphine oxide, tris(2,4-dimethylbenzoyl)phosphine oxide, tris(2-methoxybenzoyl)phosphine oxide, and salts of these (for example, sodium salts, potassium salts, ammonium salts). Preferred among these (bis)acylphosphine oxides are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the α-aminoacetophenones include 2-benzyl-2-dimethylamino-1-(4-morpholinophenyl)-1-butanone, 2-benzyl-2-diethylamino-1-(4-morpholinophenyl)-1-butanone, 2-benzyl-2-dimethylamino-1-(4-morpholinophenyl)-1-propanone, 2-benzyl-2-diethylamino-1-(4-morpholinophenyl)-1-propanone, 2-benzyl-2-dimethylamino-1-(4-morpholinophenyl)-1-pentanone, and 2-benzyl-2-diethylamino-1-(4-morpholinophenyl)-1-pentanone.

The photopolymerization initiator may be used alone, or two or more thereof may be used in combination.

The content of the photopolymerization initiator is not particularly limited. However, in view of the curability and other properties of the dental curable composition obtained, the content of the photopolymerization initiator is preferably 0.001 to 10 parts by mass, more preferably 0.005 to 5 parts by mass, even more preferably 0.01 to 3 parts by mass with respect to total 100 parts by mass of the polymerizable monomer components.

For enhanced photocurability, the photopolymerization initiator may be used with polymerization accelerators for use with photopolymerization initiators. Examples of such polymerization accelerators for photopolymerization initiators include tertiary amines, aldehydes, thiol compounds, and triazine compounds substituted with a trihalomethyl group.

Examples of the tertiary amines include:
aromatic tertiary amines such as N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, n-butoxyethyl 4-(N,N-dimethylamino)benzoate, 2-(methacryloyloxy)ethyl 4-(N,N-dimethylamino)benzoate, ethyl 4-(N,N-dimethylamino)benzoate, butyl 4-(N,N-dimethylamino)benzoate, and 4-(N,N-dimethylamino)benzophenone; and
aliphatic tertiary amines such as trimethylamine, triethylamine, N-methyldiethanolamine, N-ethyldiethanolamine, N-n-butyldiethanolamine, N-lauryldiethanolamine, triethanolamine, 2-(dimethylamino)ethyl (meth)acrylate, N-methyldiethanolamine di(meth)acrylate, N-ethyldiethanolamine di(meth)acrylate, triethanolamine mono(meth)acrylate, triethanolamine di(meth)acrylate, and triethanolamine tri(meth)acrylate.

Examples of the aldehydes include terephthalaldehyde, and benzaldehyde derivatives.

Examples of the benzaldehyde derivatives include dimethylaminobenzaldehyde, p-methoxybenzaldehyde, p-ethoxybenzaldehyde, and p-n-octyloxybenzaldehyde.

Examples of the thiol compounds include 3-mercaptopropyltrimethoxysilane, 2-mercaptobenzoxazole, decanethiol, and thiobenzoic acid.

The triazine compounds substituted with a trihalomethyl group may be any known compounds, provided that it is an s-triazine compound with at least one trihalomethyl group such as a trichloromethyl group, and a tribromomethyl group.

The polymerization accelerators for photopolymerization initiators may be used alone, or two or more thereof may be used in combination. The content of polymerization accelerators for photopolymerization initiators is not particularly limited. However, in view of the curability and other properties of the dental curable composition obtained, the content of polymerization accelerators for photopolymerization initiators is preferably 0.01 to 2 parts by mass, more preferably 0.02 to 1.5 parts by mass, even more preferably 0.05 to 0.8 parts by mass with respect to total 100 parts by mass of the polymerizable monomer components. When the content of polymerization accelerators for photopolymerization initiators is at or above these lower limits, it is easier for photopolymerization to sufficiently proceed. When the content of polymerization accelerators for photopolymerization initiators is at or below the foregoing upper limits, it is easier to control discoloration of the cured product of the dental curable composition within an acceptable range.

A dental curable composition of the present invention may additionally comprise a fluorine-ion releasing substance. By incorporating a fluorine-ion releasing substance, a dental cement can be obtained that can impart acid resistance to tooth structure.

Examples of the fluorine-ion releasing substance include fluorine-ion releasing polymers, such as copolymers of methyl methacrylate and methacrylic acid fluoride; hydrofluorides of primary, secondary, or tertiary amines of aliphatic or alicyclic nature, such as cetylamine hydrofluoride, cyclohexylamine hydrofluoride, diisobutylamine hydrofluoride, and triethylamine trihydrofluoride; and metal fluorides such as sodium fluoride, potassium fluoride, sodium monofluorophosphate, lithium fluoride, and ytterbium fluoride. The fluorine-ion releasing substance may be used alone, or two or more thereof may be used in combination.

A dental curable composition of the present invention may comprise a pH adjuster. The pH adjuster is used to adjust and stabilize the pH of a dental curable composition of the present invention.

The pH adjuster is not particularly limited, as long as the present invention can exhibit its effects. Preferred for use are acids such as lactic acid, succinic acid, gluconic acid, citric acid, phosphoric acid, and carbonic acid, and salts of these.

Examples of the phosphates include:
alkali metal salts of phosphate, such as trisodium phosphate, disodium hydrogen phosphate, sodium dihydrogen phosphate, tripotassium phosphate, dipotassium hydrogen phosphate, and potassium dihydrogen phosphate;
alkali metal salts of alkyl phosphate, alkali metal salts of hydrogen phosphate, or alkali metal salts of dihydrogen phosphate, such as sodium dodecylphosphate, sodium glycerophosphate, and disodium glycerophosphate; and
alkali-earth metal salts of phosphate, alkali-earth metal salts of hydrogen phosphate, or alkali-earth metal salts of dihydrogen phosphate, such as tricalcium phosphate, calcium hydrogen phosphate, and calcium dihydrogen phosphate.

Other examples are trimagnesium phosphate, and magnesium hydrogen phosphate. Preferred for use among these are disodium hydrogen phosphate, sodium dodecyl phosphate, sodium glycerophosphate, and disodium glycerophosphate.

In a dental curable composition of the present invention, it is also possible to incorporate additives such as pH adjusters, polymerization inhibitors, ultraviolet absorbers, solvents (for example, water, organic solvent), thickeners, colorants, antimicrobial agents, and fragrances in at least one of the first and second agents, provided that such additives do not hinder the effectiveness of the present invention. These may be incorporated alone, or two or more thereof may be incorporated in combination. Examples of the polymerization inhibitors include hydroquinone, hydroquinone monomethyl ether, dibutyl hydroquinone, dibutyl hydroquinone monomethyl ether, t-butylcatechol, 2-t-butyl-4,6-dimethylphenol, 2,6-di-t-butylphenol, and 2,6-di-t-butyl-4-methylphenol.

The content of polymerization inhibitor is preferably 0.001 to 1.0 part by mass with respect to total 100 parts by mass of polymerizable monomer components in the dental curable composition.

In certain embodiments, the content of a solvent (for example, water, organic solvent) in the paste-form two-pack type dental curable composition is preferably less than 1 mass%, more preferably less than 0.1 mass%, even more preferably less than 0.01 mass% in a total amount of the paste-form two-pack type dental curable composition.

In a dental curable composition of the present invention, it is preferable that the variation (absolute difference) in working time after 3 weeks of storage at 60°C from immediately after production, relative to the working time upon production, remain preferably within 3 minutes, more preferably within 2 minutes, even more preferably within 1 minute. The method of measurement of working time is as described in the EXAMPLES section below.

A dental curable composition of the present invention can also be suitably used as a self-adhesive composition. A dental curable composition of the present invention can be suitably used in applications, for example, such as dental resin cements, and composite resins for dental abutment construction.

The present invention encompasses embodiments combining the foregoing features in various ways within the technical idea of the present invention, provided that the present invention can exhibit its effects.

### EXAMPLES

The following describes the present invention in greater detail using Examples and Comparative Examples. It is to be noted, however, that the present invention is not limited to the following. The abbreviations and symbols used hereafter are as follows. The compounds and fillers used in the following Examples and Comparative Examples are commercially available products, except where the production methods are specifically described.

### [Acidic group-containing polymerizable monomer (A)]

MDP: 10-methacryloyloxydecyl dihydrogen phosphate

### [Acidic group-free polymerizable monomer (B)]

Bis-GMA: 2,2-bis[4-(2-hydroxy-3-methacryloyloxypropoxy)phenyl]propane
D2.6E: 2,2-bis(4-methacryloyloxypolyethoxyphenyl)propane (with an average of 2.6 moles of ethoxy group added)
TEGDMA: triethylene glycol dimethacrylate
HEMA: 2-hydroxyethyl methacrylate

### [Organic peroxide (C)]

THP: 1,1,3,3-tetramethylbutyl hydroperoxide
CHP: cumene hydroperoxide

### [Nitrogen atom-containing ligand (D)]

TEEDA: N,N,N',N'-tetraethylethylenediamine
PMDETA: N,N,N,N',N",N"-pentamethyldiethylenetriamine
TMPDA: N,N,N',N'-tetramethylpropylenediamine
HMTETA: N,N-bis(2-dimethylaminoethyl)-N,N'-dimethylethylenediamine
TMTACTD: 1,4,8,11-tetramethyl-1,4,8,11-tetraazacyclotetradecane
Me₆TREN: tris[2-(dimethylamino)ethyl]amine
DPP: 2,6-bis(1-pyrazole)-pyridine

### [Filler (E)]

Filler 1: silane-treated silica

A commercially available product (a fine spherical silica powder treated with methacrylsilane, manufactured by Admatechs under the trade name Admanano^{®} YA010C-SM1; average primary particle diameter: 10 nm, BET specific surface area: 300 m²/g) was used without modification.

### Filler 2: silane-treated silica-coated ytterbium fluoride

A commercially available product (SG-YBF100WSCMP10, manufactured by Sukgyung AT; average particle diameter: 110 nm, spherical in shape, refractive index: 1.53) was used without modification.

### Filler 3: silane-treated barium glass

A barium glass (manufactured by Esstech, product code E-3000) was pulverized with a ball mill to obtain a barium glass powder. The pulverized barium glass powder had an average particle diameter of 2.4 µm as measured by volume with a laser diffraction particle size distribution analyzer (Model SALD-2300, manufactured by Shimadzu Corporation). One-hundred parts by mass of the pulverized barium glass powder was surface treated with three parts by mass of γ-methacryloyloxypropyltrimethoxysilane by an ordinary method to obtain a silane-treated barium glass powder.

### Filler 4: alumina

A commercially available product (aluminum oxide manufactured by Nippon Aerosil Co., Ltd. under the trade name AEROXIDE^{®} Alu C; average particle diameter: 13 nm) was used without modification.

### [Thiourea compound (F)]

6MPyTU: N-(6-methyl-2-pyridyl)-thiourea
ATU: N-acetylthiourea
PyTU: 1-(2-pyridyl)-2-thiourea

### [Transition metal compound]

CuA: copper(II) acetate
Cu(acac)₂: copper(II) acetylacetonate

### [Ascorbic acid compound]

ANa: sodium L-ascorbate

### [Polymerization inhibitor]

BHT: 2,6-di-t-butyl-4-methylphenol

### (Examples 1 to 12 and Comparative Examples 1 to 6)

The components, excluding the fillers, listed in Tables 1 and 2 were mixed at ordinary temperature to prepare a uniform liquid component. Subsequently, this liquid component was kneaded with the fillers to prepare dental curable compositions of Examples 1 to 7, 9 to 12, and Comparative Examples 1 to 6. In Example 8, the components, excluding the fillers and ascorbic acid compound, were mixed at ordinary temperature to prepare a uniform liquid component. This liquid component was then kneaded with the ascorbic acid compound and fillers to prepare a dental curable composition. Immediately following their production, these dental curable compositions were utilized to measure their working time at 23°C, as well as bond strength to bovine dentin, according to the methods described below. The 23°C working time, bond strength to bovine dentin, and paste solidification were also measured after a storage period of 3 weeks at 60°C. Tables 1 and 2 present the formulation ratios (parts by mass) and the test results for these dental curable compositions. It is noted that the dental curable compositions of Comparative Examples 2, 4, and 6 were not measurable after 3 weeks of storage at 60°C because the first agent solidified during the 3-week storage period at 60°C.

### [23°C Working Time of Dental Curable Compositions]

The first agent and second agent were mixed at a 1:1 mass ratio within a thermostatic chamber set at 23°C. These were thoroughly mixed with a spatula to form a single agent. Using a thermocouple (manufactured by Okazaki Manufacturing Company) linked to a recorder (manufactured by Yokogawa Electric Corporation), the duration from the moment of mixing until the temperature began to rise due to the onset of paste's curing (referred to as working time) was measured. The working time represents the average of measured values from five test samples. A working time ranging from 2 to 8 minutes is considered suitable for practical use.

The first agent and second agent of the two-pack type dental curable composition of the present invention were filled into the compartments of SA Luting^{®} Multi (manufactured by Kuraray Noritake Dental Inc.), with 4 grams in each compartment. This composition was used for the measurement of working time conducted in the same fashion after storing the composition in a dry atmosphere at 60°C for 3 weeks.

### [Bond Strength of Dental Curable Composition to Bovine Dentin]

The labial surface of a bovine mandibular incisor was ground with #80 silicon carbide paper (manufactured by Nihon Kenshi Co., Ltd.) under running water to expose a flat dentin surface. The exposed flat surface was then polished with #1000 silicon carbide paper (manufactured by Nihon Kenshi Co., Ltd.) under running water. After polishing, the surface was dried by blowing air and removing the surface water. After drying, an about 150 µm-thick adhesive tape having a 3 mm circular hole was attached to the dried smooth dentin surface to define a bonding area.

The first agent and second agent from the dental curable composition of each Example and Comparative Example were taken in equal amounts. These were kneaded for a duration of 10 seconds, and the resulting mixture was applied onto one end surface (with a circular cross section) of a cylindrical stainless steel rod (7 mm in diameter, 2.5 cm in length).

Subsequently, the end surface with the dental curable composition was placed on the smooth surface (adherend) within the circular hole, with the center of the circular hole approximately aligned with the center of the cylindrical stainless steel rod. The cylindrical stainless steel rod was then pressed perpendicularly against the smooth surface to effect bonding and prepare a test sample. A total of five test samples were prepared. These test samples were left at 25°C for 30 minutes, and immersed in distilled water. The test samples were then left for 24 hours in the distilled water, inside a thermostatic chamber held at 37°C. After this 24-hour period at 37°C, the test samples were measured for their tensile bond strength to bovine dentin.

The tensile bond strength was measured using a universal testing machine (manufactured by Shimadzu Corporation, Autograph AG-I 100kN) with the crosshead speed set at 2 mm/min. The table presents the tensile bond strength to dentin as an average of measured tensile bond strength values from five test samples after a 24-hour period at 37°C. Separately, the first agent and second agent of the two-pack type dental curable composition of the present invention were filled into the compartments of SA Luting^{®} Multi (manufactured by Kuraray Noritake Dental Inc.), with 4 grams in each compartment. After being stored in a dry atmosphere at 60°C for 3 weeks, the composition was used for the measurement of bond strength conducted in the same fashion as in the measurement after 24-hour storage at 37°C.

### [Solidification of Dental Curable Composition (Paste)]

The first agent and second agent of the dental curable composition of the present invention were filled into the compartments of SA Luting^{®} Multi (manufactured by Kuraray Noritake Dental Inc.), with 4 grams in each compartment. This composition was then stored in a dry atmosphere at 60°C for 3 weeks, and the presence or absence of a cured product was examined for both the first and second agents. The assessment categorized the presence of a cured product as "solidification", and the absence of a cured product as "no solidification". The presence or absence of solidification was examined for five samples, and the occurrence of solidification was noted when the presence of a cured product was confirmed in at least one of the samples.

**[Table 1]**

| Components (parts by mass) | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 |
|---|---|---|---|---|---|---|---|---|---|---|---|---|---|---|
| First agent | Acidic group-containing polymerizable monomer (A) | MDP | 25 | 25 | 25 | 25 | 25 | 25 | 25 | 25 | 25 | 15 | 15 | 15 |
| | Acidic group-free polymerizable monomer (B) | Bis-GMA | 30 | 20 | 20 | 20 | 20 | 20 | 20 | 20 | 20 | 20 | 20 | 20 |
| | | D2.6E | 20 | 30 | 30 | 30 | 30 | 30 | 30 | 30 | 30 | 30 | 30 | 30 |
| | | TEGDMA | 25 | 25 | 25 | 25 | 25 | 25 | 25 | 25 | 25 | 20 | 20 | 20 |
| | | HEMA | | | | | | | | | | 15 | 15 | 15 |
| | Organic peroxide (C) | THP | 4 | 4 | 4 | 4 | 4 | 4 | 4 | 4 | | | | |
| | | CHP | | | | | | | | | 4 | 4 | 4 | 4 |
| | Nitrogen atom-containing ligand (D) | TEEDA | 2 | | | | | | | | | | | |
| | | PMDETA | | 2 | | | | | | 2 | 2 | 2 | 2 | 2 |
| | | TMPDA | | | 2 | | | | | | | | | |
| | | HMTETA | | | | 2 | | | | | | | | |
| | | TMTACTD | | | | | 2 | | | | | | | |
| | | Me₆TREN | | | | | | 2 | | | | | | |
| | | DPP | | | | | | | 2 | | | | | |
| | Polymerization inhibitor | BHT | 0.2 | 0.2 | 0.2 | 0.2 | 0.2 | 0.2 | 0.2 | 0.2 | 0.2 | 0.2 | 0.2 | 0.2 |
| | Filler (E): Filler 1 | Silane-treated silica | 185 | 185 | 185 | 185 | 185 | 185 | 185 | 185 | 185 | 185 | 185 | 185 |
| | Filler (E): Filler 2 | Silica-coated ytterbium fluoride | 50 | 50 | 50 | 50 | 50 | 50 | 50 | 50 | 50 | 50 | 50 | 50 |
| Second agent | Acidic group-free polymerizable monomer (B) | D2.6E | 35 | 35 | 35 | 35 | 35 | 35 | 35 | 35 | 35 | 40 | 40 | 40 |
| | | Bis-GMA | 30 | 30 | 30 | 30 | 30 | 30 | 30 | 30 | 30 | 30 | 30 | 30 |
| | | HEMA | 15 | 15 | 15 | 15 | 15 | 15 | 15 | 15 | 15 | | | |
| | | TEGDMA | 20 | 20 | 20 | 20 | 20 | 20 | 20 | 20 | 20 | 30 | 30 | 30 |
| | Thiourea compound (F) | 6MPyTU | 1 | 1 | 1 | 1 | 1 | 1 | 1 | 1 | | | | |
| | | ATU | | | | | | | | | | 1 | 1 | |
| | | PvTU | | | | | | | | | 1 | | | 1 |
| | Transition metal compound | CuA | | | | | | | | | | | | |
| | | Cu(acac)₂ | | | | | | | | | | | 0.01 | |
| | Ascorbic acid compound | ANa | | | | | | | | 3 | | | | |
| | Polymerization inhibitor | BHT | 0.05 | 0.05 | 0.05 | 0.05 | 0.05 | 0.05 | 0.05 | 0.05 | 0.05 | 0.05 | 0.05 | 0.05 |
| | Filler (E): Filler 3 | Silane-treated barium glass | 280 | 280 | 280 | 280 | 280 | 280 | 280 | 280 | 280 | 280 | 280 | 280 |
| | Filler (E): Filler 4 | Alumina | 16 | 16 | 16 | 16 | 16 | 16 | 16 | 16 | 16 | 16 | 16 | 16 |
| Performance | Immediately after preparation | 23°C working time (min) | 3.5 | 3.7 | 4.5 | 4.2 | 3.2 | 3.6 | 4.1 | 2.2 | 3.4 | 5.1 | 3.2 | 5.3 |
| | | Tensile bond strength to dentin (MPa) | 8.8 | 9.5 | 8.6 | 9.1 | 9.5 | 10.2 | 8.7 | 10.6 | 9.2 | 7.9 | 8.1 | 106 |
| | Accelerated temperature samples (60°C, 3 weeks) | 23°C working time (min) | 4.5 | 4.5 | 3.8 | 3.4 | 3.9 | 40 | 50 | 4.4 | 4.3 | 5.9 | 3.7 | 60 |
| | | Tensile bond strength to dentin (MPa) | 7.9 | 8.5 | 7.9 | 8.6 | 8.3 | 9.9 | 8.1 | 10.2 | 8.2 | 7.6 | 7.8 | 102 |
| | | Solidification of first agent paste | Absent | Absent | Absent | Absent | Absent | Absent | Absent | Absent | Absent | Absent | Absent | Absent |
| | | Solidification of second agent paste | Absent | Absent | Absent | Absent | Absent | Absent | Absent | Absent | Absent | Absent | Absent | Absent |

**[Table 2]**

| Components (parts by mass) | | | Com. Ex. 1 | Com. Ex. 2 | Com. Ex. 3 | Com. Ex. 4 | Com. Ex. 5 | Com. Ex. 6 |
|---|---|---|---|---|---|---|---|---|
| First agent | Acidic group-containing polymerizable monomer (A) | MDP | 25 | 15 | 25 | 15 | 25 | 15 |
| | Acidic group-free polymerizable monomer (B) | Bis-GMA | 20 | 20 | 20 | 20 | 30 | 20 |
| | | D2.6E | 30 | 30 | 30 | 30 | 20 | 30 |
| | | TEGDMA | 25 | 20 | 25 | 20 | 25 | 20 |
| | | HEMA | | 15 | | 15 | | 15 |
| | Organic peroxide (C) | THP | 4 | | 4 | | | |
| | | CHP | | 4 | | 4 | 4 | 4 |
| | Nitrogen atom-containing ligand (D) | TEEDA | | | | | | |
| | | PMDETA | | | | | | |
| | | TMPDA | | | | | | |
| | | HMTETA | | | | | | |
| | | TMTACTD | | | | | | |
| | | Me₆TREN | | | | | | |
| | | DPP | | | | | | |
| | Polymerization inhibitor | BHT | 0.2 | 0.2 | 0.2 | 0.2 | 0.2 | 0.2 |
| | Filler (E): Filler 1 | Silane-treated silica | 185 | 185 | 185 | 185 | 185 | 185 |
| | Filler (E): Filler 2 | Silica-coated ytterbium fluoride | 50 | 50 | 50 | 50 | 50 | 50 |
| Second agent | Acidic group-free polymerizable monomer (B) | D2.6E | 35 | 40 | 35 | 40 | 35 | 40 |
| | | Bis-GMA | 30 | 30 | 30 | 30 | 30 | 30 |
| | | HEMA | 15 | | 15 | | 15 | |
| | | TEGDMA | 20 | 30 | 20 | 30 | 20 | 30 |
| | Thiourea compound (F) | 6MPyTU | 1 | | 1 | | | |
| | | ATU | | 1 | | 1 | | |
| | | PvTU | | | | | 1 | 1 |
| | Transition metal compound | CuA | | | | | | |
| | | Cu(acac)₂ | | | 0.01 | 0.01 | | |
| | Ascorbic acid compound | ANa | | | | | | |
| | Polymerization inhibitor | BHT | 0.05 | 0.05 | 0.05 | 0.05 | 0.05 | 0.05 |
| | Filler (E): Filler 3 | Silane-treated barium glass | 280 | 280 | 280 | 280 | 280 | 280 |
| | Filler (E): Filler 4 | Alumina | 16 | 16 | 16 | 16 | 16 | 16 |
| Performance | Immediately after preparation | 23°C working time (min) | 3.0 | 4.7 | 1.6 | 2.1 | 2.5 | 55 |
| | | Tensile bond strength to dentin (MPa) | 7.9 | 6.5 | 7.5 | 8.2 | 7.3 | 6.2 |
| | Accelerated temperature samples (60°C, 3 weeks) | 23°C working time (min) | 16.2 | - | 12.5 | - | 109 | - |
| | | Tensile bond strength to dentin (MPa) | 3.1 | - | 2.6 | - | 3.3 | - |
| | | Solidification of first agent paste | Absent | Present | Absent | Present | Absent | Present |
| | | Solidification of second agent paste | Absent | Absent | Absent | Absent | Absent | Absent |

As shown in Table 1, the dental curable compositions according to the present invention (Examples 1 to 12) demonstrated an adequate working time at 23°C immediately after preparation, exhibiting outstanding bond strength to bovine dentin. There was no solidification noted in the paste after 3 weeks of storage at 60°C, and the 23°C working time and the bond strength to bovine dentin showed small changes after storage, compared to their values immediately after production.

In contrast, as shown in Table 2, the pastes of the dental curable compositions with no nitrogen atom-containing ligand (D) in Comparative Examples 1, 3, and 5 exhibited substantial changes in 23°C working time after 3 weeks of storage at 60°C, compared to the values immediately after preparation, suggesting significant variability in working time during extended storage periods. Additionally, these pastes displayed inadequate bond strength to bovine dentin after 3 weeks of storage at 60°C.

In the dental curable compositions of Comparative Examples 2, 4, and 6 with no nitrogen atom-containing ligand (D), the first agent underwent solidification during the 3-week storage period at 60°C, demonstrating a high risk of solidification over extended storage.

### INDUSTRIAL APPLICABILITY

A dental curable composition of the present invention can be suitably used in applications such as luting of dental prostheses, such as crowns, inlays, and bridges, to tooth structures, and construction of abutments in dental treatments.

## Claims

1. A paste-form two-pack type dental curable composition comprising:
a first agent that comprises an acidic group-containing polymerizable monomer (A), an acidic group-free polymerizable monomer (B), an organic peroxide (C), a nitrogen atom-containing ligand (D), and a filler (E); and
a second agent that comprises an acidic group-free polymerizable monomer (B), a thiourea compound (F), and a filler (E).

2. The paste-form two-pack type dental curable composition according to claim 1, wherein the nitrogen atom-containing ligand (D) comprises a ligand having two or more nitrogen atoms within the molecule.

3. The paste-form two-pack type dental curable composition according to claim 1 or 2, wherein:
the nitrogen atom-containing ligand (D) is at least one compound selected from the group consisting of a compound represented by general formula (1), a compound represented by general formula (2), and a polydentate ligand (3) containing a nitrogen-containing heterocyclic ring, and
the polydentate ligand (3) is a bidentate or higher dentate ligand compound that comprises a heterocyclic ring containing a five-membered or six-membered ring having a nitrogen atom, and has two or more nitrogen atoms within the molecule,
R₁R₂N-X₁-NR₃R₄ (1)
wherein R₁ to R₄ each independently represent an optionally substituted alkyl group, and X₁ represents an optionally substituted divalent aliphatic group, 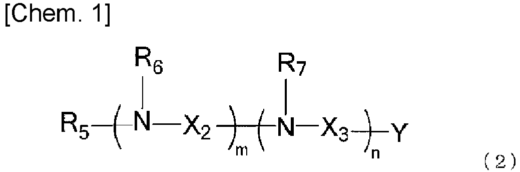 wherein R₅, R₆, and R₇ each independently represent an optionally substituted alkyl group, X₂ and X₃ each independently represent an optionally substituted divalent aliphatic group that may contain an oxygen atom and/or a nitrogen atom, m and n each independently represent an integer of 1 or more, Y represents an optionally substituted monoalkylamino group or dialkylamino group, and any two or more of R₅, R₆, R₇, and Y may together form a ring, and R₆, R₇, X₂, and X₃ may be the same or different when R₆, R₇, X₂, and X₃ are plural.

4. The paste-form two-pack type dental curable composition according to claim 3, wherein the nitrogen atom-containing ligand (D) is at least one compound selected from the group consisting of a compound represented by general formula (1), and a compound represented by general formula (2).

5. The paste-form two-pack type dental curable composition according to any one of claims 1 to 3, wherein the nitrogen atom-containing ligand (D) is at least one compound selected from the group consisting of N,N,N',N'-tetraethylethylenediamine, N,N,N,N',N",N"-pentamethyldiethylenetriamine, N,N,N',N'-tetramethylpropylenediamine, N,N-bis(2-dimethylaminoethyl)-N,N'-dimethylethylenediamine, 1,4,8,11-tetramethyl-1,4,8,11-tetraazacyclotetradecane, tris[2-(dimethylamino)ethyl]amine, and 2,6-bis(1-pyrazole)-pyridine.

6. The paste-form two-pack type dental curable composition according to any one of claims 1 to 3, wherein the nitrogen atom-containing ligand (D) is at least one compound selected from the group consisting of N,N,N,N',N",N"-pentamethyldiethylenetriamine, N,N,N',N'-tetramethylpropylenediamine, N,N-bis(2-dimethylaminoethyl)-N,N'-dimethylethylenediamine, and tris[2-(dimethylamino)ethyl]amine.

7. The paste-form two-pack type dental curable composition according to any one of claims 1 to 6, wherein the ratio of the acidic group-containing polymerizable monomer (A) and the nitrogen atom-containing ligand (D) in the first agent is 40:1 to 2:1 (mass ratio).

8. The paste-form two-pack type dental curable composition according to any one of claims 1 to 7, which is a self-adhesive composition.
